# EUROPEAN PATENT SPECIFICATION

(11) **EP 4 134 071 B1**
(45) Date of publication and mention of the grant of the patent: **29.04.2026**
(21) Application number: 21918101.3
(22) Date of filing: 28.12.2021
(51) Int. Cl.: A61K 9/48, A61K 47/36, A23P 10/30

(54) **SOFT CAPSULE SHELL AND SOFT CAPSULE**
WEICHKAPSELHÜLLE UND WEICHKAPSEL
ENVELOPPE DE CAPSULE MOLLE ET CAPSULE MOLLE

(30) Priority: 28.06.2021 CN 202110719246
(43) Date of publication of application: 15.02.2023
(73) Proprietor: Sirio Pharma Co., Ltd., Shantou, Guangdong 515000 (CN)
(72) Inventor: CHEN, Jiewei, Guangdong 515000 (CN); LI, Xufa, Guangdong 515000 (CN)
(74) Representative: Cabinet Becker et Associés
(86) International application number: PCT/CN2021/142080
(87) International publication number: WO 2023/273255

(56) References cited:
- EP-A1- 1 792 939
- EP-A1- 2 815 744
- EP-A1- 3 785 707
- EP-A1- 4 134 072
- WO-A1-2019/208668
- CN-A- 1 663 989
- CN-A- 113 398 088
- JP-A- 2009 040 716
- US-A1- 2016 136 101
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631 - 662, XP009102859, ISSN: 0193-2691, DOI: 10.1081/DIS-120015368

## Description

### Technical Field

The invention relates to the field of food or medicines, in particular to a soft capsule shell and a soft capsule.

### Background of the invention

Traditional enteric soft capsules are mainly based on coating. Soft capsules are first produced and then coated to obtain the enteric effect. Preparation of enteric soft capsules mainly employs one-step shaping in recent studies, that is, adding enteric materials during the process of dissolving, shaping capsules by compression, and finally drying. It is in an uncoated form that does not require a separate coating. The non-coated enteric soft capsule can improve the preservation durability of the coated soft capsule, and it has simplified process, improved efficiency, and reduced cost compared with the traditional preparation process.

The non-coated enteric soft capsules can be prepared using compositions of gelatin and pectin, compositions of carrageenan and acrylic resin, and compositions of high and low acyl gellan gums and starch. For example, patent CN106456558A proposes a soft capsule prepared from a gelatin and a low methoxy pectin. However, the reaction of monovalent and divalent cations with pectin makes the enteric solubility of the soft capsules poor, and long-term storage makes the enteric solubility of the soft capsules further inferior. Also, gelatin is prone to crosslinking due to aging or due to reaction with compounds such as aldehydes, which is thus resistant to acid in simulated gastric fluid, but fails to rupture and disintegrate in simulated intestinal fluid. Furthermore, gelatin is mainly derived from cows and pigs, and vegetarians and certain religions do not consume animal-derived gelatin, which further limits the application of gelatin. Patent CN105263462A proposes a soft capsule prepared from film materials composed of a carrageenan composition, a methacrylic acid copolymer, a modified starch, a plasticizer, etc. EP3010493A1 proposes a film composition of gastric acid-resistant soft capsules, which is obtained by mixing a high acyl gellan gum, a low acyl gellan gum, a starch and a plasticizer. However, such gel compounded by high and low acyl gellan gum is easy to pre-gel during the process of preparation of ribbons for soft capsules, which makes the ribbons rough and uneven, poor in formability, and easy to leak oil.

Patent Application EP 4 134 072 discloses a soft capsule shell intended to be disintegrated into the stomach. The soft shell capsule is prepared from a film-forming composition comprising 2-5 wt% of a first gelling agent, 10-35 wt% of a plasticizer, 18-35 wt% of gelatinizable starch, 4-7 wt% of granular starch and 35-55 wt% of water.

Patent application EP 2 815 744 discloses gastro-restistant soft shell capsule comprising (i) high acyl gellan gum having more than 40% acetyl and more than 45% glyceryl residual substituents per repeat unit, (ii) at least one starch, and (iii) at least one plasticizer.

Patent application EP 3 785 707 relates to soft capsule film composition comprising: (A) a native gellan gum, (B) one or more hydrophilic polysaccharides, (C) a starch or a modified starch, (D) a plasticizer, and (E) water.

Patent application EP 1 792 939 relates to a composition comprising a gelling starch, a non-gelling starch, a high acyl gellan gum, a low acid gellan gum, and a plasticizer.

In conclusion, the existing gelatin system of enteric soft capsules is limited in applications, and the scope of application of the contents is narrow. The synthetic resin in the carrageenan system cannot be added to soft capsules in some areas, and the gellan gum system has poor formability and is easy to leak oil etc. Those skilled in the art need enteric soft capsules that solve the above technical problems.

### Summary of the Invention

The present invention is based in part on the inventors' findings from long-term work that the state of the starch in the soft capsule shell is a granular starch or a segment after the granular starch is broken, wherein the average particle size of the starch granules is 10 microns -50 microns. When the content of starch granules in the soft capsule shell is 0% to 3%, the network structure of the gellan gum in the soft capsule shell can support the mechanical force generated by the hardening of the soft capsule in simulated gastric fluid, to ensure that it will not rupture in simulated gastric fluid, and rupture in simulated intestinal fluid, finally achieving the enteric effect of soft capsules. The inventors found that 1.5 wt% of the gellan gum with a viscosity of 10 mPa.s-60Pa.s at 90°C in combination with starch may prepare a soft capsule with good healing properties, high rupture resistance and enteric release, which is suitable for industrial production.

In one aspect, the present invention provides a soft capsule shell prepared from a film-forming composition comprising 1.5 to 5 wt% of a first gelling agent, 30 to 40 wt% of a starch, 10 to 25 wt% of a plasticizer, and 35 to 55 wt% of water, wherein the first gelling agent is a gellan gum with the following properties: the gellan gum solution obtained by dissolving 1.5 wt% of the gellan gum in water at 90°C for 30 minutes has a viscosity ranging from 10 to 60 mPa.s as measured by a rotational viscometer, wherein the soft capsule shell comprises 0 to 3 wt% of starch granules, and the starch granules are those with an average particle size of 10 microns to 50 microns. The soft capsule shell herein is an enteric soft capsule shell.

In one embodiment, the starch is one or more of a hydroxypropyl starch, an oxidized starch, or an oxidized hydroxypropyl starch.

In one embodiment, the starch is one or more of a hydroxypropyl starch, an oxidized starch, or an oxidized hydroxypropyl starch and not more than 2 wt% of one or more of an acid-treated starch, an acetate starch, a hydroxypropyl distarch phosphate, a starch phosphate, an acetylated distarch phosphate, an acetylated oxidized starch, an acetylated distarch adipate, or a pregelatinized starch.

In one embodiment, the weight ratio of the gellan gum to the starch ranges from 0.03 to 0.2.

In one embodiment, the plasticizer is selected from one or a combination of glycerol, sorbitol, maltitol, erythritol, xylitol, crystalline fructose, trehalose or glucose.

In one embodiment, the film-forming composition further comprises a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum, preferably the content of the second gelling agent ranges from 0.2 wt% to 5 wt%.

In one embodiment, the soft capsule shell is prepared by a method comprising a) adding a first gelling agent to a plasticizer, stirring them homogeneously, followed by adding the mixture into water, and heating them at a temperature between 60 and 98°C while stirring until the first gelling agent dissolves; b) adding the starch, and heating them at a temperature between 60 and 98°C while stirring until the starch dissolves; c) removing air bubbles to obtain a gel mass; and d) encapsulating and drying.

In one embodiment, the step a) further comprises adding a second gelling agent, the step a) is a step for adding the first gelling agent and the second gelling agent to the plasticizer, stirring them homogeneously, followed by adding the mixture into water, and heating them at a temperature between 60 and 98°C while stirring until the first gelling agent and the second gelling agent dissolve.

In another aspect, the present invention provides a soft capsule comprising the soft capsule shell of the present invention and a filling. In one embodiment, the soft capsule is an enteric soft capsule. In one embodiment, the soft capsule is an enteric soft capsule shell without enteric coating.

In another aspect, the present invention provides use of the soft capsule shell or soft capsule of the present invention in food products, nutraceutical food products and drug products.

In another aspect, the present invention provides use of the film-forming composition in the preparation of an enteric soft capsule shell, wherein the film-forming composition comprises 1.5 to 5 wt% of a first gelling agent, 30 to 40 wt% of a starch, 10 to 25 wt% of a plasticizer, and 35 to 55 wt% of water, wherein the first gelling agent is a gellan gum with the following properties: the gellan gum solution obtained by dissolving 1.5 wt% of the gellan gum in water at 90°C for 30 minutes has a viscosity ranging from 10 to 60 mPa.s as measured by a rotational viscometer, wherein the enteric soft capsule shell comprises 0 to 3 wt% of starch granules, and the starch granules are those with an average particle diameter of 10 microns to 50 microns.

In one embodiment, the soft capsule shell is prepared by a method comprising a) adding a first gelling agent to a plasticizer, stirring them homogeneously, followed by adding the mixture into water, and heating them at a temperature between 60 and 98°C while stirring until the first gelling agent dissolves; b) adding the starch, and heating them at a temperature between 60 and 98°C while stirring until the starch dissolves; c) removing air bubbles to obtain a gel mass; and d) encapsulating and drying.

In one embodiment, the step a) further comprises adding a second gelling agent, the step a) is a step for adding the first gelling agent and the second gelling agent to the plasticizer, stirring them homogeneously, followed by adding the mixture into water, and heating them at a temperature between 60 and 98°C while stirring until the first gelling agent and the second gelling agent dissolve.

In one embodiment, the starch is one or more of a hydroxypropyl starch, an oxidized starch, or an oxidized hydroxypropyl starch; or the starch is one or more of a hydroxypropyl starch, an oxidized starch, or an oxidized hydroxypropyl starch and not more than 2 wt% of one or more of an acid-treated starch, an acetate starch, a hydroxypropyl distarch phosphate, a starch phosphate, an acetylated distarch phosphate, an acetylated distarch adipate, an acetylated oxidized starch, or a pregelatinized starch.

The embodiments of the soft capsule shell may also be applicable to the aspect of the use.

The present invention also provides a film-forming composition for preparing soft capsule shells, which comprises 1.5 to 5 wt% of a first gelling agent, 30 to 40 wt% of a starch, 10 to 25 wt% of a plasticizer and 35 to 55wt% of water, wherein the first gelling agent is a gellan gum with the following properties: the gellan gum solution obtained by dissolving 1.5 wt% of the gellan gum in water at 90°C for 30 minutes has a viscosity ranging from 10 to 60 mPa.s as measured by a rotational viscometer.

The embodiments of the soft capsule shell may also be applicable to the aspect of the film-forming composition.

In another aspect, the present invention provides a soft capsule comprising the soft capsule shell of the present invention and a filling. In one embodiment, the soft capsule is an enteric soft capsule. In one embodiment, the filling is a filling applicable to the intestine, such as a drug or a nutritional supplement.

In the present invention, the preparation method of the soft capsule shell may not comprise the step of coating.

In another aspect, the present invention provides use of the film-forming composition, the soft capsule shell or the soft capsule of the present invention in food products, nutraceutical products and drug products. In one embodiment, the food, nutraceutical or drug products are enterically absorbed or administered.

In one aspect, the present invention provides a method of enteral delivery using the soft capsule shell or soft capsule of the present invention. The present invention also provides use of the soft capsule shell or soft capsule of the present invention in the preparation of a medicament used in the intestine.

In one embodiment, the film-forming composition or soft capsule shell of the present invention does not comprise gelatin. In one embodiment, the film-forming composition or soft capsule shell of the present invention does not comprise pectin. That is, the soft capsule shells of the present invention have excellent soft capsule performance indexes and achieve enteric effect without gelatin and/or pectin.

The soft capsule of the present invention prepared by employing a composition of a gellan gum with a particular viscosity and a starch has strength, toughness and seam adhesive property for shaping that fully meet the requirements for industrial production of soft capsules. The soft capsule product with enteric effect is directly prepared without coating, which can be used as an alternative in enteric soft capsule technology and be promoted and applied in the global market.

### Brief Description of Figures

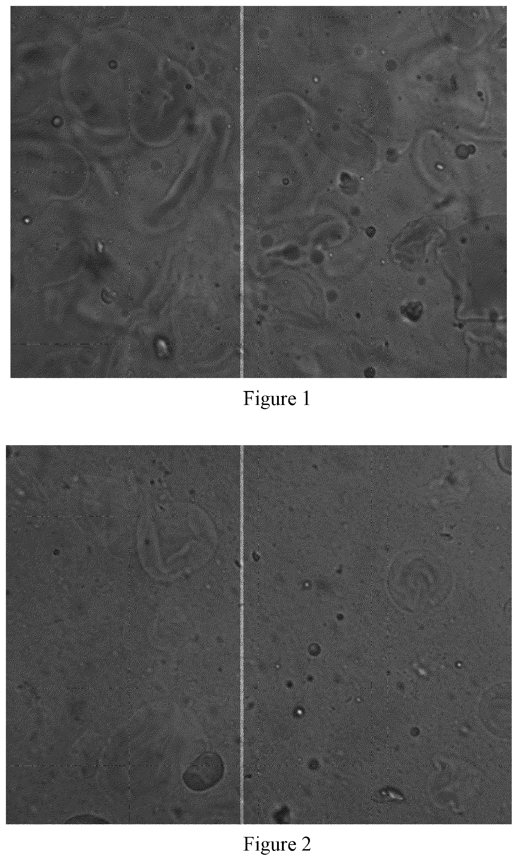

Fig. 1 shows the optical micrograph of a film, which is prepared by heating the gel mass prepared in Example 11 that is placed between two glass slides at 95°C under pressure to form a film, prior to cooling to form a capsule shell, which is then stained by dripping iodine solution thereon. The magnification factor of the image is 600 (shown is the detail image of the film with a width of 266.1 µm). The starch granules are separated by centrifugation, which account for 5% of the soft capsule shell by mass. It can be seen from the figure that there are plurality of starch granules distributed in the capsule shell.
Fig. 2 shows the optical micrograph of a film, which is prepared by heating the gel mass prepared in Example 4 that is placed between two glass slides at 95°C under pressure to form a film, prior to cooling to form a capsule shell, which is then stained by dripping iodine solution thereon. The magnification factor of the image is 600 (shown is the detail image of the film with a width of 266.1 µm). The starch granules are separated by centrifugation, which account for 3% of the soft capsule shell by mass. It can be seen from the figure that there are fewer starch granules distributed in the capsule shell.

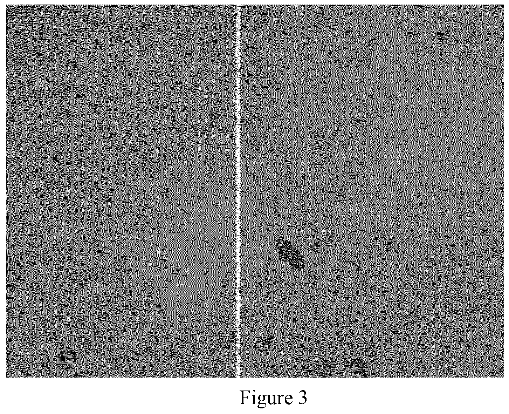

Fig. 3 shows the optical micrograph of a film, which is prepared by heating the gel mass prepared in Example 9 that is placed between two glass slides at 95°C under pressure to form a film, prior to cooling to form a capsule shell, which is then stained by dripping iodine solution thereon. The magnification factor of the image is 600 (shown is the detail image of the film with a width of 266.1 µm). The starch granules are separated by centrifugation, which account for 0% of the soft capsule shell by mass. It can be seen from the figure that there is no starch granule distributed in the capsule shell. In capsule shell they are all in the form of granular broken starch and gellan gum dispersed phase.

### Detailed Description of the invention

The following is provided to further illustrate the present invention.

The present invention provides a soft capsule shell prepared from a film-forming composition of the present invention. The film-forming composition of the present invention comprises a first gelling agent. The content of the first gelling agent may be 1.5 wt% to 5 wt%, for example, 2 wt%, 3 wt%, 4 wt% or 5 wt%. Preferably, the content of the first gelling agent may be 2 wt% to 5 wt%, for example, 2.5 to 5 wt%. The first gelling agent may be a gellan gum. The first gelling agent may be a gellan gum with the following properties: the gellan gum solution obtained by dissolving 1.5 wt% of the gellan gum in water at 90°C for 30 minutes has a viscosity ranging from 10 to 60 mPa.s, for example, a viscosity of 12, 15, 20, 30, 35, 40, 45, 50 or 55 mPa.s, as measured by a rotational viscometer. Preferably, the viscosity ranges from 30 to 50 mPa.s. In one embodiment, the gellan gum is a gellan gum with single gelling temperature. The gellan gum with suitable viscosity can show good film-forming properties during the film-forming process of the gel mass. When the viscosity of the gellan gum used is too low, the amount of gellan gum used must be increased to prepare a film with qualified strength, additionally with low gelling rate of the ribbon, poor film-forming performance, and insufficient viscosity which makes the capsules with poor performance at the seam, poor in healing, and serious oil leakage; when the viscosity of gellan gum used is too high, the prepared gel mass is too thick, and easy to pre-gel during the process, which leads to rough ribbon, poor film-forming performance, and poor film viscosity, which makes the capsules not heal well, resulting in oil leakage of the capsules; the gellan gum with too high and too low viscosity fails to prepare a qualified ribbon for use in the compression and formation of a soft capsule. Herein, the inventors achieved the present invention using 1.5 to 5 wt% of the gellan gum in a starch film-forming composition, wherein the gellan gum solution obtained by dissolving 1.5 wt% of the gellan gum in water at 90°C for 30 minutes has a viscosity ranging from 10 to 60 mPa.s, as measured by a rotational viscometer.

The film-forming composition comprises a plasticizer. The content of plasticizer may be 10-25 wt%, for example 13 wt%, 14 wt%, 15 wt%, 16 wt% or 20 wt%. The plasticizer may be selected from one or a combination of glycerol, sorbitol, maltitol, erythritol, xylitol, crystalline fructose, trehalose or glucose.

The film-forming composition comprises a starch. The content of starch may be 30 to 40 wt%, for example 30 wt%, 35 wt% or 40 wt% of a starch. The starch can be one or a combination of a native starch or a modified starch. The native starch can be selected from one or a combination of a waxy corn starch, a pea starch, a corn starch, a potato starch, and a tapioca starch. The modified starch can be selected from one or a combination of an acid-treated starch, a hydroxypropyl starch, an oxidized starch, an acetate starch, an oxidized hydroxypropyl starch, a hydroxypropyl distarch phosphate, a starch phosphate, an acetylated distarch phosphate, an acetylated distarch adipate, a pregelatinized starch, a dextrin or a maltodextrin. In one embodiment, the weight ratio of gellan gum to starch ranges from 0.03 to 0.2. In one embodiment, the weight ratio of gellan gum to starch ranges from 0.04 to 0.17. For example, the weight ratio of gellan gum to starch is 0.04, 0.09, 0.1, 0.13 or 0.17. Preferably, the starch is one or more of a hydroxypropyl starch, an oxidized starch or an oxidized hydroxypropyl starch. Alternatively, the starch comprises a gelatinizable starch and a granular starch. The gelatinizable starch refers to a starch whose granules are broken or dissolved after complete gelatinization of starch, for example selected from a waxy corn starch, a tapioca starch, a hydroxypropyl starch, an oxidized starch, an oxidized hydroxypropyl starch, a dextrin and a maltodextrin. The granular starch refers to a starch that does not gelatinize completely and whose granules are not broken after water swelling, which is selected from: a native starch: a pea starch, a corn starch, a potato starch; a modified starch: an acid-treated starch, an acetate starch, a hydroxypropyl distarch phosphate, a starch phosphate, an acetylated distarch phosphate, an acetylated distarch adipate, an acetylated oxidized starch, a pregelatinized starch. In one embodiment, the content of the gelatinizable starch is 30-40 wt% of the starch, for example 30 wt%, 32 wt%, 35 wt% or 40 wt%. In one embodiment, the content of the granular starch is not more than 2 wt%, for example 1 wt%, 1.5 wt% or 1.7 wt%.

The film-forming composition comprises water. The content of water may be 35 wt% to 55 wt%, for example 40 wt%, 45 wt%, 50 wt% or 55 wt%.

The film-forming composition may further comprise a second gelling agent. The content of the second gelling agent may be 0.2 wt% to 5 wt%, for example 0.25 wt%, 1 wt%, 2 wt%, 3 wt% or 4 wt%. The second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum.

The present invention provides a soft capsule comprising a soft capsule shell of the present invention and a filling. The filling can comprise one or a combination of various animal and vegetable fat, or suspensions, emulsions, semi-solids prepared from various solid functional ingredients and suitable excipients for soft capsules, or solid preparations (such as granules, microcapsule, powder, plain tablet, capsule) prepared from solid functional ingredients and suitable excipients. The state of starch in the soft capsule shell may be starch granules (average size of starch granules ≥ 10µm), a granular broken starch (average size of starch granules < 10µm) or segments of starch granules after breaking up, in which the content of starch granules is 0 to 3 wt%, e.g. 1 or 2 wt% of the total amount of the soft capsule shell.

The present invention also provides a method for preparing the soft capsule shell of the present invention, which comprises a) adding a first gelling agent to a plasticizer, stirring them homogeneously, followed by adding the mixture to water, and heating them at a temperature between 60 and 98°C while stirring until the first gelling agent dissolves; b) adding the starch, and heating them at a temperature between 60 and 98°C while stirring until the starch dissolves; c) removing air bubbles to obtain a gel mass; and d) encapsulating and drying. In one embodiment, the step a) further comprises adding a second gelling agent, the step a) is a step for adding the first gelling agent and the second gelling agent to the plasticizer, stirring them homogeneously, followed by adding the mixture to water, and heating them at a temperature between 60 and 98°C while stirring until the first gelling agent and the second gelling agent dissolve. Encapsulation can be carried out as follows: a soft capsule production line is employed and the gel mass is transported to the spreader box of the soft capsule encapsulation machine, the gel mass is cooled on the surface of a rotating drum to form a ribbon, then soft capsules are formed by extrusion and encapsulation when injecting the filling, and can be further shaped in a rotating cage device. Drying can be carried out as follows: the capsules are further dried after forming or shaping, preferably dried until the moisture content of the capsule shell is 8 to 25%.

The present invention also provides use of the starch film-forming composition, soft capsule shell or soft capsule of the present invention in food products, nutraceutical food products, drug products and cosmetics.

The advantages of the present invention comprise:
1. The gellan gum with a particular viscosity is selected for preparation of the soft capsule by examining the viscosity of the gellan gum in the invention. The film-forming composition of the present invention forms films with desired strength and toughness, by using the gellan gum with a particular viscosity, which in combination with starch is controlled within a certain range. By using in the soft capsule, the film-forming composition of the present invention is obviously superior to those in the prior art in terms of the strength, toughness and seam adhesive property for shaping of ribbon, which can fully meet the requirements for industrial production of soft capsules, and the regulations of the disintegration time limit of enteric soft capsules, and can be used as an alternative in non-coated enteric soft capsule technology.
2. It is found in the present invention that the disintegration and rupture of the soft capsule in medium water and simulated gastric fluid are related to the particle size and content of the starch granules by observing the starch granules in the capsule shell.
3. It is found in the present invention that the film-forming composition comprising the combination of particular types and content of starch (such as gelatinizable starch and no more than 2 wt% of granular starch) can achieve unexpected effects on the comprehensive evaluation of soft capsules and enteric solubility, compared with the film-forming compositions comprising a single type of starch, for example hydroxypropyl starch.

### Examples

### Methods used in the examples

1. In order to characterize the starch granules in the capsule shell, the following methods are used to measure the size and content of the granule:
   1) Size of starch granule. The gel mass prepared by mixing and heating the film-forming composition is placed between two glass slides, and the gel mass is heated and pressurized to form a film at 95°C. Such film is stained by dropwise adding iodine solution after cooling, and a polarizing microscope is used for taking pictures. Multiple observation areas are randomly selected to take pictures. After calibrating the scale according to the magnification, the particle size of each starch granule in the photographed area is measured, and the particle size of starch is represented as arithmetic mean value.
   2) Content of starch granule. 100 mg of dried soft capsule shells are dissolved with 15 g of deionized water at 75°C for 30 minutes, with stirring for several times during this period to completely dissolve the capsule shells. The turbid solution of the dissolved soft capsule shells is then centrifuged at 4000 rpm for 15min. After centrifugation under these conditions, the starch granules are basically completely in the centrifugal sediment, and the upper liquid is removed. After the bottom settlings are fully dried, the content of starch granule is represented as the content of sediment, and the proportion of starch granule in the soft capsule shells is calculated by mass.

In order to better illustrate the effects of the present invention, the following indicators of a soft capsule are used for evaluation and description.

### (1) Formation

1) Indicators of the strength (F) and toughness (T) of a ribbon. A texture analyzer is used with a spherical probe and puncture mode selected under test speed of 1.0 mm/s. Record the force when the ribbon ruptures, and the greater the force, the better the strength of the ribbon. And record the corresponding distance (mm) upon rupture of the ribbon, and the longer the distance, the better the toughness of the ribbon.
2) Indicator of the adhesive property at a seam. The capsule is cut at a position other than the seam and emptied by extrusion of filling. Then, a ring with two seams at the middle of the capsule is cut off perpendicularly to the seam, which is placed on a glass slide with the two seams perpendicular to the glass slide. The thicknesses of the two seams and the capsule shell are measured under a microscope. The ratio P (%) of the thickness of the thinnest seam to that of the capsule shell is calculated.

**Table 1 Evaluation standard of the indicators of the strength, toughness and adhesive property at a seam of the soft capsule ribbon.**

| Evaluation Indicators | Weig ht | Score | | | | | |
|---|---|---|---|---|---|---|---|
| | | 5 points | 4 points | 3 points | 2 points | 1 point | 0 point |
| Indicator of the strength of the ribbon (F, in N) | 20% | F ≥ 1.51N | 1.17N ≤ F<1.51N | 0.83N ≤ F<1.17 | 0.49N ≤ F<0.83N | F<0.49 N | failure to form a ribbon |
| Indicator of the toughness of the ribbon (T, in mm) | 20% | T ≥ 10mm | 8mm ≤ T<10mm | 6mm ≤ T<8mm | 4mm ≤ T<6mm | T<4mm | failure to form a ribbon |
| Indicator of the adhesive property at a seam (P, in %) | 60% | P ≥ 60% | 50% ≤ P < 60% | 40%≤P< 50% | 30%≤ P<40% | 0<P<30 % | failure to be shaped |

The feasibility of soft capsule production and formation is comprehensively evaluated using the ribbon strength (F), toughness (T) and the adhesive property at a seam (P) as indicators, wherein the comprehensive evaluation is obtained by adding up 20% of score for the ribbon strength, 20% of score for toughness and 60% of score for adhesive property at a seam. The total score for the comprehensive evaluation is 5 points, and the higher the score, the better the comprehensive performance. Both indicators of ribbon strength and toughness are respectively ≥ 3 points and the indicator of the adhesive property at a seam is ≥2 points, and the comprehensive evaluation must be ≥2.4 to meet the industrial production of soft capsules. The total score for the comprehensive evaluation of the three indicators is 5 points, and the higher the score, the better the comprehensive performance.

### 2. Assay of disintegration time limit of enteric soft capsules

For the present invention, soft capsules must meet:
1) In accordance with USP <2040> Disintegration and Dissolution of Dietary Supplements, the delayed-release (enteric-coated) soft-shell capsules are subjected to a disintegration test. No capsule shows signs of disintegration or rupture permitting the escape of the content in simulated gastric fluid for at least 60 minutes, followed by disintegration in simulated intestinal fluid over no more than 60 minutes.
2) Using the European Pharmacopoeia, the disintegration test EP 2.9.1 as defined in soft capsules, no capsule shows signs of disintegration or rupture permitting the escape of the content in simulated gastric fluid for at least 120 minutes. Subsequent disintegration in phosphate buffered solution at pH 6.8 occurs in no more than 60 minutes.

### 3. Viscosity determination

Gellan gum is a microbial metabolic gum. The gellan gums produced under different process conditions vary in rheological properties and gel properties. The inventors dissolved 1.5 wt% of the gellan gum in water at 90°C for 30min to obtain a gellan gum solution, and studied the viscosity of commercially available gellan gum by using a rotational viscometer:
Gellan Gum A (Viscosity 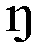 =12.2 mPa.s)
Gellan Gum B (Viscosity 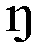 =30.8 mPa.s)
Gellan Gum C (Viscosity 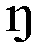 =20.4 mPa.s)
Gellan Gum D (Viscosity 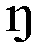 =48.8 mPa.s)
Gellan Gum E (Viscosity 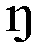 =43.7 mPa.s)
Gellan Gum F (Viscosity 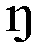 =52.8 mPa.s)
Gellan Gum G (Viscosity 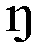 =65.2 mPa.s)
Gellan Gum H (Viscosity 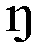 =7.8 mPa.s).

The above gellan gums are all gellan gums with a single gelling temperature.

4. The method for preparing a soft capsule comprising:
1) Gel mass preparation: A) firstly premixing and dispersing a gellan gum and a plasticizer homogeneously, adding the mixture into appropriate amount of water while stirring, and then heating them at a temperature between 60 and 98°C while stirring until the gellan gum dissolves; if an edible gum other than gellan gum is included, firstly premixing and dispersing the gellan gum, the edible gum and the plasticizer homogeneously, adding the mixture into water while stirring, and then heating them at a temperature between 60 and 98°C while stirring until the gellan gum and the edible gum dissolve; B) adding a starch, and continuing to heat at a temperature between 60 and 98°C while stirring until the starch dissolves; and C) removing air bubbles to obtain a gel mass.
2) Encapsulation: the soft capsule production line is employed and the gel mass is transported to a spreader box of a soft capsule encapsulation machine, the gel mass is cooled on the surface of a rotating drum to form a ribbon, and then a soft capsule is formed by extrusion and encapsulation when injecting the filling, and can be further shaped by a rotating cage device.
3) Drying: the capsule is dried after forming or shaping, until the moisture content of the shell is 8-25%.

### Examples 1-8

Soft capsules were prepared with the components and contents described in Table 2, and tested and scored. The measurement results of determination are shown in Table 2.

According to Table 2, it can be seen that the ribbons formed by employing the combination of gellan gum with a particular viscosity and starch in Examples 1-8 have a good strength, toughness and seam adhesive property upon formation; with the same contents of components as those of the film-forming compositions of the present invention in Control Examples 1-2, the ribbons formed by employing the combination of gellan gum with higher viscosity and starch in Control Example 1 have poor strength, some toughness, but average adhesive property upon formation; the ribbons formed by employing the combination of gellan gum with lower viscosity and starch in Control Example 2 have great viscosity, poor toughness of the ribbon, and are unable to be shaped by compression to make soft capsules.

### Examples 9-23

Soft capsules were prepared with the components and contents described in Tables 3 and 4, and tested and scored. The measurement results of determination are shown in Tables 3 and 4. The soft capsules prepared in Example 9, Example 10 and the above Example 4 can all achieve enteric effect. Due to the addition of granular starch, the comprehensive evaluation of the soft capsules made in Example 10 and the above Example 4 becomes better than that in Example 9. However, when the granular starch is increased to 3%, the resulting soft capsules are disintegrated in the stomach. Therefore, the addition of less than 2% (e.g., 1.7%) of cross-linked starch, such as hydroxypropyl distarch phosphate, achieves unexpected effects on comprehensive evaluation and enteric solubility of soft capsules.

**Table 2**

| Formula composition | Example 1 | Example 2 | Example 3 | Example 4 | Example 5 | Example 6 | Example 7 | Example 8 | Control Example 1 | Control Example 2 | |
|---|---|---|---|---|---|---|---|---|---|---|---|
| Gellan gum A | 3.0% | / | / | / | / | / | / | / | / | / | |
| Gellan gum B | / | 3.0% | / | / | / | / | / | / | / | / | |
| Gellan gum C | / | / | 3.0% | / | / | / | / | / | / | / | |
| Gellan gum D | / | / | / | 3.0% | / | / | 1.5% | 5.0% | / | / | |
| Gellan gum E | / | / | / | / | 3.0% | / | / | / | / | / | |
| Gellan gum F | / | / | / | / | / | 3.0% | / | / | / | / | |
| Gellan gum G | / | / | / | / | / | / | / | / | 3.0% | / | |
| Gellan gum H | | | | | | | | | | 3.0% | |
| Hydroxypropyl starch | 32.3% | 32.3% | 32.3% | 32.3% | 32.3% | 32.3% | 39% | 28.3% | 32.3% | 32.3% | |
| Hydroxypropyl distarch phosphate | 1.7% | 1.7% | 1.7% | 1.7% | 1.7% | 1.7% | 1% | 1.7% | 1.7% | 1.7% | |
| Glycerol | 20.0% | 20.0% | 20.0% | 20.0% | 20.0% | 20.0% | 20.0% | 20.0% | 20.0% | 20.0% | |
| Water | 43.0% | 43.0% | 43.0% | 43.0% | 43.0% | 43.0% | 38.5% | 45.0% | 43.0% | 43.0% | |
| Ratio of the gellan gum to the starch | 0.09 | 0.09 | 0.09 | 0.09 | 0.09 | 0.09 | 0.04 | 0.17 | 0.09 | 0.09 | |
| Strength of the ribbon | 3 | 4 | 4 | 4 | 4 | 4 | 3 | 5 | 2 | 1 | |
| Toughness of the ribbon | 3 | 4 | 4 | 5 | 4 | 3 | 3 | 5 | 3 | 2 | |
| Seam adhesive property upon formation | 4 | 5 | 3 | 5 | 5 | 3 | 4 | 2 | 2 | 1 | |
| Comprehensive evaluation | | 3.6 | 4.6 | 3.4 | 4.8 | 4.6 | 3.2 | 3.6 | 3.2 | 2.2 | 1.2 |
| Average particle size of starch granules/µm | | 42 | 43 | 43 | 45 | 45 | 46 | 48 | 47 | 45 | 45 |
| The percentage of starch granules in the capsular shells by mass | | 3% | 3% | 3% | 3% | 3% | 3% | 1.8% | 3% | 3% | 3% |
| United States Pharmacopoei a <2040> | 1h simulated gastric fluid | √ | √ | √ | √ | √ | √ | √ | √ | × | not applicabl e |
| | 1h simulated intestinal fluid | × | × | × | × | × | × | × | × | / | not applicabl e |
| European Pharmacopoei a <2.9.1> | 2h simulated gastric fluid | √ | √ | √ | √ | √ | √ | √ | √ | × | not applicabl e |
| | 1h simulated intestinal fluid | × | × | × | × | × | × | × | × | / | not applicabl e |

| | | | | | | | | | | | |
|---|---|---|---|---|---|---|---|---|---|---|---|
| √ indicates no rupture ×indicates rupture | | | | | | | | | | | |

**Table 3**

| Formula composition | Examp le 9 | Examp le 10 | Examp le 4 | Examp le 11 | Examp le 12 | Examp le 13 | Examp le 14 | Examp le 15 | Examp le 16 | Examp le 17 | Examp le 18 | Examp le 19 | |
|---|---|---|---|---|---|---|---|---|---|---|---|---|---|
| Gellan gum D | 3.0% | 3.0% | 3.0% | 3.0% | 3.0% | 3.0% | 3.0% | 3.0% | 3.0% | 3.0% | 3.0% | 3.0% | |
| Hydroxypropyl starch | 34.0% | 33.4% | 32.3% | 31% | 14% | / | 17% | 23.8% | / | 23.8% | 23.8% | 23.8% | |
| Hydroxypropyl distarch phosphate | / | 0.6% | 1.7% | 3% | 20% | / | / | / | / | / | / | / | |
| Oxidized starch | / | / | / | / | / | 34% | 17% | / | / | / | / | / | |
| Acetate starch | / | / | / | / | / | / | / | 10.2% | / | / | / | / | |
| Oxidized hydroxypropyl starch | / | / | / | / | / | / | / | / | 34% | / | / | / | |
| Acetylated oxidized starch | / | / | / | / | / | / | / | / | / | 10.2% | / | / | |
| Acetylated distarch phosphate | / | / | / | / | / | / | / | / | / | / | 10.2% | / | |
| Acid modified starch | / | / | / | / | / | / | / | / | / | / | / | 10.2% | |
| Glycerol | 20.0% | 20.0% | 20.0% | 20.0% | 20.0% | 20.0% | 20.0% | 20.0% | 20.0% | 20.0% | 20.0% | 20.0% | |
| Water | 43.0% | 43.0% | 43.0% | 43.0% | 43.0% | 43.0% | 43.0% | 43.0% | 43.0% | 43.0% | 43.0% | 43.0% | |
| Ratio of the gellan gum to the starch | 0.09 | 0.09 | 0.09 | 0.09 | 0.09 | 0.09 | 0.09 | 0.09 | 0.09 | 0.09 | 0.09 | 0.09 | |
| Strength of the ribbon | 3 | 4 | 4 | 5 | 5 | 3 | 4 | 5 | 3 | 5 | 5 | 5 | |
| Toughness of the ribbon | 3 | 4 | 5 | 5 | 2 | 3 | 3 | 5 | 3 | 5 | 5 | 5 | |
| Seam adhesive property upon formation | 4 | 5 | 5 | 5 | 1 | 3 | 3 | 5 | 3 | 5 | 5 | 5 | |
| Comprehensive evaluation | 3.6 | 4.6 | 4.8 | 5 | 2 | 3 | 3.2 | 5 | 3 | 5 | 5 | 5 | |
| Average particle size of starch granules/µm | 1.5 | 45 | 45 | 45 | 45 | 1.2 | 1.5 | 15 | 1.5 | 30 | 55 | 33 | |
| The percentage of starch granules in the capsular shells by mass | | 0% | 1.0% | 3.0% | 5.0% | 35.3% | 0% | 0% | 17.2% | 0% | 18.9% | 19.2% | 18.7% |
| United States Pharmacopoei a <2040> | 1h simulated gastric fluid | √ | √ | √ | × | × | √ | √ | × | √ | × | × | × |
| | 1h simulated intestinal fluid | × | × | × | / | / | × | × | / | × | / | / | / |
| European Pharmacopoei a <2.9.1> | 2h simulated gastric fluid | √ | √ | √ | × | × | √ | √ | × | √ | × | × | × |
| | 1h simulated intestinal fluid | × | × | × | / | / | × | × | / | × | / | / | / |

| | | | | | | | | | | | | | |
|---|---|---|---|---|---|---|---|---|---|---|---|---|---|
| √ indicates no rupture ×indicates rupture | | | | | | | | | | | | | |

**Table 4**

| Formula composition | Example 9 | Example 4 | Example 20 | Example 21 | Example 22 | Example 23 | |
|---|---|---|---|---|---|---|---|
| Gellan gum D | 3.0% | 3.0% | 3.0% | 3.0% | 3.0% | 3.0% | |
| Hydroxypropyl starch | 34.0% | 32.3% | 32.3% | 32.3% | 32.3% | 32.3% | |
| Hydroxypropyl distarch phosphate | / | 1.7% | / | / | / | / | |
| Acetate starch | | / | / | 1.7% | / | / | / |
| Acetylated oxidized starch | | / | / | / | 1.7% | / | / |
| Acetylated distarch phosphate | | / | / | / | / | 1.7% | / |
| Acid modified starch | | / | / | / | / | / | 1.7% |
| Glycerol | | 20.0% | 20.0% | 20.0% | 20.0% | 20.0% | 20.0% |
| Water | | 43.0% | 43.0% | 43.0% | 43.0% | 43.0% | 43.0% |
| Ratio of the gellan gum to the starch | | 0.09 | 0.09 | 0.09 | 0.09 | 0.09 | 0.09 |
| Strength of the ribbon | | 3 | 4 | 5 | 5 | 5 | 5 |
| Toughness of the ribbon | | 3 | 5 | 5 | 5 | 5 | 5 |
| Seam adhesive property upon formation | | 4 | 5 | 4 | 4 | 4 | 4 |
| Comprehensive evaluation | | 3.6 | 4.8 | 4.4 | 4.4 | 4.4 | 4.4 |
| Average particle size of starch granules/µm | | 1.5 | 45 | 15 | 30 | 40 | 33 |
| The percentage of starch granules in the capsular shells by mass | | 0% | 3% | 3% | 3% | 3% | 3% |
| United States Pharmacopoeia<2040> | 1h simulated gastric fluid | | | | | | |
| | 1h simulated intestinal fluid | × | × | × | × | × | × |
| European Pharmacopoeia<2.9.1> | 2h simulated gastric fluid | | | | | | |
| | 1h simulated intestinal fluid | × | × | × | × | × | × |

| | | | | | | | |
|---|---|---|---|---|---|---|---|
| √ indicates no rupture ×indicates rupture | | | | | | | |

Examples 9-23 illustrate that the technical effect of soft capsules prepared by using the gellan gum with a particular viscosity in combination with various types of starch is better than that of the prior art, and can achieve the enteric effect.

The size of starch granules in the soft capsule shell can be observed by a polarizing microscope with 600 times magnification. During the process of dissolving of starch and gellan gum, after the starch is heated in an aqueous medium, some starch granules are first further dissolved due to water swelling, allowing the swollen starch granules to break to form smaller starch granules, which are further dissolved while stirring, resulting in the starch finally present in the gel mass in a solution state along with the separation and dissolution of starch molecular segments. The gel mass is first cooled and then formed into ribbons during the shaping process in an encapsulation machine, in which the starch forms broken starch or interpenetrating network structure with the molecular segments of gellan gum, from the relatively free molecular segments in the gel mass by rapidly cooling down during the temperature drop. If the starch cannot be further swelled, broken, and finally dissolved due to the intermolecular force of the starch itself after the starch granules water swelling in the process of gel dissolution, the starch will exist in the form of swollen granules in the gelling solution. At this time, the starch granules show no cross extinction phenomenon under a polarizing microscope. The gel mass at this time is cooled and shaped during the forming process of the encapsulation machine, and the starch in the gel mass still exists in the capsule shell of the soft capsule in the form of swollen granules. At this time, the gellan gum molecules cannot penetrate inside the starch granules to form a structure of interpenetrating network with the starch molecular chain segments. The reason why non-coated enteric soft capsules can resist gastric acid erosion in simulated gastric fluid is that: when the segments of gellan gum molecules are in the simulated gastric fluid environment, the hydrogen ions and water molecules in hydrochloric acid can combine with carboxylate radicals on the double helix molecular chain of gellan gum to form hydrogen bonds, which allows the whole double helix molecules cross-link with the adjoining double helix molecules of gellan gum through hydrogen bonding to form a network structure. However, during the disintegration in water or simulated gastric fluid, soft capsules prepared from gellan gum and starch swell with volume increased by 2-5 times, and, along with the swelling, the swollen soft capsules become brittle. The smaller the proportion of starch granules in the soft capsule shell, the greater the strength of the gellan gum network structure. Eventually, the binding force of the gellan gum network structure is greater than the failure stress for the soft capsule to swell and become brittle, so that no capsule shows signs of disintegration or rupture permitting the escape of the content in simulated gastric fluid. Therefore, when the binding force generated by the cross-linking reaction of the gellan gum double helix molecular chains is greater than the failure stress for swelling and brittleness, no capsule shows signs of disintegration or rupture permitting the escape of the content in simulated gastric fluid; when the binding force generated by the cross-linking reaction of gellan gum double helix molecular chains is smaller than the failure stress for swelling and brittleness, the soft capsule will rupture in simulated gastric fluid, resulting in the failure of the soft capsule to achieve enteric effect.

### Examples 24-31

Soft capsules were prepared with the components and contents described in Table 5, and tested and scored. The measurement results of determination are shown in Table 5.

**Table 5**

| Formula composition | Example 4 | Example 24 | Example 25 | Example 26 | Example 27 | Example 28 | Example 29 | Example 30 | Example 31 |
|---|---|---|---|---|---|---|---|---|---|
| Gellan gum D | 3% | 2.5% | 2.5% | 2.5% | 2.5% | 2.5% | 2.5% | 2.5% | 2.5% |
| Agar | / | 0.5% | / | / | / | / | / | / | / |
| Locust bean gum | / | / | 0.5% | / | / | / | / | / | / |
| Guar gum | / | / | / | 0.5% | / | / | / | / | / |
| Konjac glucomannan | / | / | / | / | 0.5% | / | / | / | / |
| Low ester pectin | / | / | / | / | / | 0.5% | / | / | / |
| Amide pectin | / | / | / | / | / | / | 0.5% | / | / |
| Carrageenan | / | / | / | / | / | / | / | 0.5% | / |
| Xanthan gum | / | / | / | / | / | / | / | / | 0.5% |
| Hydroxypropyl starch | 32.3% | 32.3% | 32.3% | 32.3% | 32.3% | 32.3% | 32.3% | 32.3% | 32.3% |
| Hydroxypropyl distarch phosphate | 1.7% | 1.7% | 1.7% | 1.7% | 1.7% | 1.7% | 1.7% | 1.7% | 1.7% |
| Glycerol | 20% | 20% | 20% | 20% | 20% | 20% | 20% | 20% | 20% |
| Water | 43% | 43% | 43% | 43% | 43% | 43% | 43% | 43% | 43% |
| Ratio of the gellan gum to the starch | 0.09 | 0.07 | 0.07 | 0.07 | 0.07 | 0.07 | 0.07 | 0.07 | 0.07 |
| Strength of the ribbon | 4 | 3 | 4 | 4 | 4 | 4 | 4 | 4 | 4 |
| Toughness of the ribbon | 5 | 4 | 4 | 4 | 5 | 5 | 5 | 4 | 4 |
| Seam adhesive property upon formation | 5 | 5 | 5 | 5 | 4 | 5 | 5 | 5 | 5 |
| Comprehensive evaluation | 4.8 | 4.4 | 4.6 | 4.6 | 4.2 | 4.8 | 4.8 | 4.6 | 4.6 |
| Average particle size of starch granules/µm | 45 | 44 | 47 | 45 | 46 | 45 | 46 | 44 | 45 |
| The percentage of starch granules in the capsular shells by mass | 3% | 3% | 3% | 3% | 3% | 3% | 3% | 3% | 3% |
| 1h simulated gastric fluid (United States Pharmacopoeia <2040>) | √ | √ | √ | √ | √ | √ | √ | √ | √ |
| 1h simulated intestinal fluid (United States Pharmacopoeia <2040>) | × | × | × | × | × | × | × | × | × |
| 2h simulated gastric fluid (European Pharmacopoeia <2.9.1>) | √ | √ | √ | √ | √ | √ | √ | √ | √ |
| 1h simulated intestinal fluid (European Pharmacopoeia <2.9.1>) | × | × | × | × | × | × | × | × | × |

| | | | | | | | | | |
|---|---|---|---|---|---|---|---|---|---|
| √ indicates no rupture ×indicates rupture | | | | | | | | | |

In Examples 25-31, the technical effect of soft capsules prepared by using the gellan gum with a particular viscosity and hydroxypropyl starch in combination with agar, locust bean gum, guar gum, konjac glucomannan, low ester pectin, amide pectin, carrageenan, and xanthan gum, respectively, is better than that of the prior art, and can achieve the enteric effect.

The present invention provides a composition of gellan gum with a particular viscosity and starch. By using the gellan gum with a certain viscosity in combination with starch under within a certain range, the composition has a good formation performance and enteric effect, can fully meet the requirements for industrial production of soft capsules, and can be used as an alternative in enteric soft capsule technology.

## Claims

1. A soft capsule shell prepared from a film-forming composition comprising 1.5 to 5 wt% of a first gelling agent, 30 to 40 wt% of a starch, 10 to 25 wt% of a plasticizer, and 35 to 55 wt% of water, wherein the first gelling agent is a gellan gum with the following properties: the gellan gum solution obtained by dissolving 1.5 wt% of the gellan gum in water at 90°C for 30 minutes has a viscosity ranging from 10 to 60 mPa.s as measured by a rotational viscometer, wherein the soft capsule shell comprises 1 to 3 wt% of starch granules, and the starch granules are starch granules with an average particle size of 10 microns to 50 microns measured by polarizing microscope; the soft capsule shell is enteric.

2. The soft capsule shell of claim 1, wherein the starch is one or more of a hydroxypropyl starch, an oxidized starch, or an oxidized hydroxypropyl starch; or the starch is (1) one or more of a hydroxypropyl starch, an oxidized starch, or an oxidized hydroxypropyl starch and (2) not more than 2 wt% of one or more of an acid-treated starch, an acetate starch, a hydroxypropyl distarch phosphate, a starch phosphate, an acetylated distarch phosphate, an acetylated oxidized starch, an acetylated distarch adipate, or a pregelatinized starch.

3. The soft capsule shell of claim 1 or 2, wherein the weight ratio of the gellan gum to the starch ranges from 0.03 to 0.2.

4. The soft capsule shell of any one of claims 1-3, wherein the plasticizer is selected from one or a combination of glycerol, sorbitol, maltitol, erythritol, xylitol, crystalline fructose, trehalose or glucose.

5. The soft capsule shell of any one of claims 1-4, wherein the film-forming composition further comprises a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum, preferably the content of the second gelling agent ranges from 0.2 wt% to 5 wt%.

6. The soft capsule shell of any one of claims 1-5, wherein the soft capsule shell is prepared by a method comprising a) adding a first gelling agent to a plasticizer, stirring them homogeneously, followed by adding the mixture into water, and heating them at a temperature between 60 and 98°C while stirring until the first gelling agent dissolves; b) adding the starch, and heating them at a temperature between 60 and 98°C while stirring until the starch dissolves; c) removing air bubbles to obtain a gel mass; and d) encapsulating and drying.

7. The soft capsule shell of claim 6, wherein the step a) further comprises adding a second gelling agent, the step a) is a step for adding the first gelling agent and the second gelling agent to the plasticizer, stirring them homogeneously, followed by adding the mixture into water, and heating them at a temperature between 60 and 98°C while stirring until the first gelling agent and the second gelling agent dissolve.

8. A soft capsule comprising the soft capsule shell of any one of claims 1-7 and a filling, preferably wherein the soft capsule shell is an enteric soft capsule shell, preferably an enteric soft capsule shell without enteric coating.

9. Use of the soft capsule shell according to any one of claims 1-7 or the soft capsule of claim 8 in food products, nutraceutical products and drug products.

10. Use of a film-forming composition in the preparation of an enteric soft capsule shell, wherein the film-forming composition comprises 1.5 to 5 wt% of a first gelling agent, 30 to 40 wt% of a starch, 10 to 25 wt% of a plasticizer, and 35 to 55 wt% of water, wherein the first gelling agent is a gellan gum with the following properties: the gellan gum solution obtained by dissolving 1.5 wt% of the gellan gum in water at 90°C for 30 minutes has a viscosity ranging from 10 to 60 mPa.s as measured by a rotational viscometer, wherein the enteric soft capsule shell comprises 1 to 3 wt% of starch granules, and the starch granules are starch granules with an average particle diameter of 10 microns to 50 microns measured by polarizing microscope;
preferably, wherein the starch is one or more of a hydroxypropyl starch, an oxidized starch, or an oxidized hydroxypropyl starch; or the starch is one or more of a hydroxypropyl starch, an oxidized starch, or an oxidized hydroxypropyl starch and not more than 2 wt% of one or more of an acid-treated starch, an acetate starch, a hydroxypropyl distarch phosphate, a starch phosphate, an acetylated distarch phosphate, an acetylated distarch adipate, an acetylated oxidized starch, or a pregelatinized starch;
preferably wherein the weight ratio of the gellan gum to the starch ranges from 0.03 to 0.2;
preferably the plasticizer is selected from one or a combination of glycerol, sorbitol, maltitol, erythritol, xylitol, crystalline fructose, trehalose or glucose;
preferably, wherein the film-forming composition further comprises a second gelling agent, preferably, the second gelling agent is selected from one or a combination of carrageenan, agar, sodium alginate, pectin, pullulan, konjac glucomannan, xanthan gum, locust bean gum, guar gum, flaxseed gum, Curdlan gum or tamarind gum, preferably the content of the second gelling agent ranges from 0.2 wt% to 5 wt%;
preferably, wherein the soft capsule shell is prepared by a method comprising a) adding a first gelling agent to a plasticizer, stirring them homogeneously, followed by adding the mixture to water, and heating them at a temperature between 60 and 98°C while stirring until the first gelling agent dissolves; b) adding the starch, and heating them at a temperature between 60 and 98°C while stirring until the starch dissolves; c) removing air bubbles to obtain a gel mass; and d) encapsulating and drying;
preferably, wherein the step a) further comprises adding a second gelling agent, and the step a) is a step for adding the first gelling agent and the second gelling agent to the plasticizer, stirring them homogeneously, followed by adding the mixture to water, and heating them at a temperature between 60 and 98°C while stirring until the first gelling agent and the second gelling agent dissolve.

11. The soft capsule shell according to any one of claims 1-7 or the soft capsule of claim 8 for use in food products, nutraceutical products and drug products.

## Patentansprüche

1. Eine Weichkapselhülle hergestellt aus einer filmbildenden Zusammensetzung umfassend 1,5 bis 5 Gew.-% eines ersten Geliermittels, 30 bis 40 Gew.-% einer Stärke, 10 bis 25 Gew.-% eines Weichmachers und 35 bis 55 Gew.-% Wasser, wobei das erste Geliermittel ein Gelangummi ist mit den folgenden Eigenschaften: Die Gellangummilösung erhalten durch Auslösen von 1,5 Gew.-% Gellangummi in Wasser bei 90°C für 30 Minuten, hat eine Viskosität von 10 bis 60 mPa s gemessen mit einem Rotationsviskosimeter, wobei die Weichkapselhülle 1 bis 3 Gew.-% Stärkekörner umfasst, und die Stärkekörner Stärkekörner mit einer durchschnittlichen Partikelgröße von 10 Mikrometer bis 50 Mikrometer gemessen mitels Polarisationsmikroskop sind; die Weichkapselhülle ist enterisch.

2. Die Weichkapselhülle gemäß Anspruch 1, wobei die Stärke eine oder mehrere aus einer Hydroxypropylstärke, einer oxidierten Stärke oder einer oxidierte Hydroxypropylstärke ist; oder die Stärke ist (1) eine oder mehrere aus einer Hydroxypropylstärke, einer oxidierten Stärke oder einer oxidierten Hydroxypropylstärke und (2) nicht mehr als 2 Gew.-% einer oder mehrerer aus einer Säure-behandelten Stärke, einer Acetat-Stärke, einer Hydroxypropyl-Distärkephosphat, einem Stärkephosphat, einem acetylierten Distärkephosphat, einer acetylierten oxidierten Stärke, einer acetylierted Distärkeadipat oder einer vorgelatinierter Stärke,

3. Die Weichkapselhülle gemäß Anspruch 1 oder 2, wobei das Gewichtsverhältnis des Gellangummis zur Stärke von 0,03 bis 0,2 reicht.

4. Die Weichkapselhülle gemäß einem der Ansprüche 1 bis 3, wobei der Weichmacher ausgewählt ist aus einem oder einer Kombination aus Glycerin, Sorbit, Maltit, Erythrit, Xylit, kristalliner Fructose, Trehalose oder Glucose.

5. Die Weichkapselhülle gemäß einem der Ansprüche 1 bis 4, wobei die filmbildende Zusammensetzung ferner ein zweites Geliermittel umfasst, bevorzugt, ist das zweite Geliermittel ausgewählt aus einem oder einer Kombination aus Carrageen, Agar, Natriumalginat, Pektin, Pullulan, Konjak-Glucomannan, Xanthangummi, Johannisbrotkernmehl, Guarkernmehl, Leinsamenmehl, Curdlan-Gummi oder Tamarindengummi, bevorzugt reicht der Gehalt des zweiten Geliermittels von 0,2 Gew.-% bis 5 Gew.-%.

6. Die Weichkapselhülle gemäß einem der Ansprüche 1 bis 5, wobei die Weichkapselhülle hergestellt wird durch ein Verfahren umfassend a) Hinzufügen eines ersten Geliermittels zu einem Weichmacher, homogenes Verrühren, gefolgt vom Einrühren der Mischung in Wasser und Erhitzen auf eine Temperatur zwischen 60 und 98 °C unter Rühren, bis sich das erste Geliermittel aufgelöst hat; b) Zugabe der Stärke und Erhitzen auf eine Temperatur zwischen 60 und 98 °C unter Rühren, bis sich die Stärke aufgelöst hat; c) Entfernen von Luftblasen, um eine Gelmasse zu erhalten; und d) Verkapseln und Trocknen.

7. Die Weichkapselhülle gemäß Anspruch 6, wobei der Schritt a) ferner das Hinzufügen eines zweiten Geliermittels, wobei der Schritt a) darin besteht, das erste Geliermittel und das zweite Geliermittel zum Weichmacher hinzuzufügen, diese homogen zu verrühren, anschließend die Mischung in Wasser zu geben und sie unter Rühren auf eine Temperatur zwischen 60 und 98 °C zu erhitzen, bis sich das erste Geliermittel und das zweite Geliermittel aufgelöst haben.

8. Eine Weichkapsel, umfassend die Weichkapselhülle gemäß einem der Ansprüche 1 bis 7 und eine Füllung, wobei die Weichkapselhülle vorzugsweise eine enterische Weichkapselhülle ist, vorzugsweise eine enterische Weichkapselhülle ohne enterische Beschichtung.

9. Verwendung der Weichkapselhülle gemäß einem der Ansprüche 1 bis 7 oder der Weichkapsel gemäß Anspruch 8 in Lebensmitteln, Nutrazeutika und Arzneimitteln.

10. Verwendung einer filmbildenden Zusammensetzung zur Herstellung einer enterischen Weichkapselhülle, wobei die filmbildende Zusammensetzung 1,5 bis 5 Gew.-% eines ersten Geliermittels, 30 bis 40 Gew.-% einer Stärke, 10 bis 25 Gew.-% eines Weichmachers und 35 bis 55 Gew.-% Wasser umfasst, wobei das erste Geliermittel ein Gellangummi mit den folgenden Eigenschaften ist: die Gellangummi-Lösung, erhalten durch Auflösen von 1,5 Gew.-% des Gellangummis in Wasser bei 90 °C für 30 Minuten, weist eine Viskosität im Bereich von 10 bis 60 mPa·s auf, gemessen mit einem Rotationsviskosimeter, wobei die enterische Weichkapselhülle 0 bis 3 Gew.-% Stärkekörner umfasst, und die Stärkekörner Stärkekörner mit einem durchschnittlichen Partikeldurchmesser von 10 Mikrometern bis 50 Mikrometern gemessen mitels Polarisationsmikroskop sind;
vorzugsweise, wobei die Stärke ist eine oder mehrere von einer Hydroxypropylstärke, einer oxidierten Stärke oder einer oxidierten Hydroxypropylstärke; oder die Stärke ist eine oder mehrere von einer Hydroxypropylstärke, einer oxidierten Stärke oder einer oxidierten Hydroxypropylstärke und nicht mehr als 2 Gew.-% einer oder mehrerer von einer säurebehandelten Stärke, einer Acetatstärke, eines Hydroxypropyl-Distärkephosphats, eines Stärkephosphats, eines acetylierten Distärkephosphats, eines acetylierten Distärkeadipats, einer acetylierten oxidierten Stärke oder einer vorgelatinierten Stärke;
wobei vorzugsweise das Gewichtsverhältnis von Gellangummi zur Stärke im Bereich von 0,03 bis 0,2 liegt;
wobei vorzugsweise der Weichmacher aus einem oder einer Kombination von Glycerin, Sorbit, Maltit, Erythrit, Xylit, kristalliner Fructose, Trehalose oder Glucose ausgewählt ist;
wobei vorzugsweise die filmbildende Zusammensetzung ferner ein zweites Geliermittel umfasst, wobei das zweite Geliermittel vorzugsweise ausgewählt ist aus einem oder einer Kombination von Carrageen, Agar, Natriumalginat, Pektin, Pullulan, Konjak-Glucomannan, Xanthangummi, Johannisbrotkernmehl, Guarkernmehl, Leinsamengummi, Curdlangummi oder Tamarindengummi, wobei der Gehalt des zweiten Geliermittels vorzugsweise im Bereich von 0,2 Gew.-% bis 5 Gew.-% liegt;
wobei vorzugsweise die Weichkapselhülle hergestellt wird durch ein Verfahren umfassend a) Zugabe eines ersten Geliermittels zu einem Weichmacher, homogenes Rühren derselben, gefolgt von Zugabe der Mischung zu Wasser und Erhitzen derselben unter Rühren auf eine Temperatur zwischen 60 und 98 °C, bis sich das erste Geliermittel aufgelöst hat; b) Zugabe der Stärke und Erhitzen derselben unter Rühren auf eine Temperatur zwischen 60 und 98 °C, bis sich die Stärke aufgelöst hat; c) Entfernen von Luftblasen, um eine Gelmasse zu erhalten; und d) Verkapseln und Trocknen;
wobei der Schritt a) vorzugsweise ferner das Hinzufügen eines zweiten Geliermittels umfasst und der Schritt a) ein Schritt ist zum Hinzufügen des ersten Geliermittels und des zweiten Geliermittels zu dem Weichmacher, zum homogenen Vermischen derselben, gefolgt vom Hinzufügen der Mischung zu Wasser und zum Erhitzen derselben unter Rühren auf eine Temperatur zwischen 60 und 98 °C, bis sich das erste Geliermittel und das zweite Geliermittel aufgelöst haben.

11. Die Weichkapselhülle gemäß einem der Ansprüche 1 bis 7 oder die Weichkapsel gemäß Anspruch 8 zur Verwendung in Lebensmitteln, Nutrazeutika und Arzneimitteln.

## Revendications

1. Enveloppe de capsule molle préparée à partir d'une composition filmogène comprenant 1,5 à 5 % en poids d'un premier agent gélifiant, 30 à 40 % en poids d'un amidon, 10 à 25 % en poids d'un plastifiant, et 35 à 55 % en poids d'eau, dans laquelle le premier agent gélifiant est une gomme gellane présentant les propriétés suivantes : la solution de gomme gellane obtenue par dissolution de 1,5 % en poids de la gomme gellane dans de l'eau à 90 °C pendant 30 minutes présente une viscosité située dans la plage allant de 10 à 60 mPa.s telle que mesurée par un viscosimètre rotatif, laquelle enveloppe de capsule molle comprend 1 à 3 % en poids de granules d'amidon, et les granules d'amidon sont des granules d'amidon présentant une taille de particule moyenne de 10 micromètres à 50 micromètres mesurée au moyen d'un microscope polarisant ; l'enveloppe de capsule molle est gastrorésistante.

2. Enveloppe de capsule molle selon la revendication 1, dans laquelle l'amidon est un ou plusieurs parmi un amidon hydroxypropylé, un amidon oxydé et un amidon hydroxypropylé oxydé ; ou l'amidon est (1) un ou plusieurs parmi un amidon hydroxypropylé, un amidon oxydé et un amidon hydroxypropylé oxydé et (2) pas plus de 2 % en poids d'un ou plusieurs parmi un amidon traité aux acides, un acétate d'amidon, un phosphate de diamidon hydroxypropylé, un phosphate d'amidon, un phosphate de diamidon acétylé, un amidon oxydé acétylé, un adipate de diamidon acétylé ou un amidon prégélatinisé.

3. Enveloppe de capsule molle selon la revendication 1 ou 2, dans laquelle le rapport en poids de la gomme gellane à l'amidon est situé dans la plage allant de 0,03 à 0,2.

4. Enveloppe de capsule molle selon l'une quelconque des revendications 1 à 3, dans laquelle le plastifiant est choisi parmi un ou une combinaison de glycérol, sorbitol, maltitol, érythritol, xylitol, fructose cristallin, tréhalose ou glucose.

5. Enveloppe de capsule molle selon l'une quelconque des revendications 1 à 4, dans laquelle la composition filmogène comprend en outre un deuxième agent gélifiant ; de préférence, le deuxième agent gélifiant est choisi parmi l'un ou une combinaison de carraghénane, agar, alginate de sodium, pectine, pullulane, glucomannane de konjac, gomme xanthane, gomme de caroube, gomme guar, gomme de lin, gomme curdlan ou gomme de tamarin ; de préférence la teneur en le deuxième agent gélifiant est située dans la plage allant de 0,2 % en poids à 5 % en poids.

6. Enveloppe de capsule molle selon l'une quelconque des revendications 1 à 5, laquelle enveloppe de capsule molle est préparée par une méthode comprenant : a) l'addition d'un premier agent gélifiant à un plastifiant, leur agitation jusqu'à homogénéité, puis l'addition du mélange dans de l'eau, et leur chauffage à une température comprise entre 60 et 98 °C sous agitation jusqu'à ce que le premier agent gélifiant se dissolve ; b) l'addition de l'amidon, et le chauffage du tout à une température comprise entre 60 et 98 °C sous agitation jusqu'à ce que l'amidon se dissolve ; c) l'élimination des bulles d'air pour que soit obtenue une masse de gel ; et d) l'encapsulation et le séchage.

7. Enveloppe de capsule molle selon la revendication 6, dans laquelle l'étape a) comprend en outre l'addition d'un deuxième agent gélifiant, l'étape a) est une étape pour ajouter le premier agent gélifiant et le deuxième agent gélifiant au plastifiant, les agiter jusqu'à homogénéité, après quoi le mélange est ajouté dans de l'eau et le tout est chauffé à une température comprise entre 60 et 98 °C sous agitation jusqu'à ce que le premier agent gélifiant et le deuxième agent gélifiant se dissolvent.

8. Capsule molle comprenant l'enveloppe de capsule molle de l'une quelconque des revendications 1 à 7 et un agent de remplissage, de préférence dans laquelle l'enveloppe de capsule molle est une enveloppe de capsule molle gastrorésistante, de préférence une enveloppe de capsule molle gastrorésistante sans enrobage entérique.

9. Utilisation de l'enveloppe de capsule molle de l'une quelconque des revendications 1 à 7 ou de la capsule molle de la revendication 8 dans des produits alimentaires, des produits nutraceutiques et des produits médicamenteux.

10. Utilisation d'une composition filmogène dans la préparation d'une enveloppe de capsule molle gastrorésistante, dans laquelle la composition filmogène comprend 1,5 à 5 % en poids d'un premier agent gélifiant, 30 à 40 % en poids d'un amidon, 10 à 25 % en poids d'un plastifiant, et 35 à 55 % en poids d'eau, dans laquelle le premier agent gélifiant est une gomme gellane présentant les propriétés suivantes : la solution de gomme gellane obtenue par dissolution de 1,5 % en poids de la gomme gellane dans de l'eau à 90 °C pendant 30 minutes présente une viscosité située dans la plage allant de 10 à 60 mPa.s telle que mesurée par un viscosimètre rotatif, dans laquelle l'enveloppe de capsule molle gastrorésistante comprend 1 à 3 % en poids de granules d'amidon, et les granules d'amidon sont des granules d'amidon présentant une granulométrie moyenne de 10 micromètres à 50 micromètres mesurée au moyen d'un microscope polarisant ;
de préférence, dans laquelle l'amidon est un ou plusieurs parmi un amidon hydroxypropylé, un amidon oxydé et un amidon hydroxypropylé oxydé ; ou l'amidon est un ou plusieurs parmi un amidon hydroxypropylé, un amidon oxydé et un amidon hydroxypropylé oxydé et pas plus de 2 % en poids d'un ou plusieurs parmi un amidon traité aux acides, un acétate d'amidon, un phosphate de diamidon hydroxypropylé, un phosphate d'amidon, un phosphate de diamidon acétylé, un adipate de diamidon acétylé, un amidon oxydé acétylé ou un amidon prégélatinisé ;
de préférence dans laquelle le rapport en poids de la gomme gellane à l'amidon est situé dans la plage allant de 0,03 à 0,2 ;
de préférence le plastifiant est choisi parmi l'un ou une combinaison de glycérol, sorbitol, maltitol, érythritol, xylitol, fructose cristallin, tréhalose ou glucose ;
de préférence dans laquelle la composition filmogène comprend en outre un deuxième agent gélifiant ; de préférence le deuxième agent gélifiant est choisi parmi l'un ou une combinaison de carraghénane, agar, alginate de sodium, pectine, pullulane, glucomannane de konjac, gomme xanthane, gomme de caroube, gomme guar, gomme de lin, gomme curdlan ou gomme de tamarin, de préférence la teneur en le deuxième agent gélifiant est située dans la plage allant de 0,2 % en poids à 5 % en poids ;
de préférence, dans laquelle l'enveloppe de capsule molle est préparée par une méthode comprenant : a) l'addition d'un premier agent gélifiant à un plastifiant, leur agitation jusqu'à homogénéité, puis l'addition du mélange dans de l'eau, et leur chauffage à une température comprise entre 60 et 98 °C sous agitation jusqu'à ce que le premier agent gélifiant se dissolve ; b) l'addition de l'amidon, et le chauffage du tout à une température comprise entre 60 et 98 °C sous agitation jusqu'à ce que l'amidon se dissolve ; c) l'élimination des bulles d'air pour que soit obtenue une masse de gel ; et d) l'encapsulation et le séchage ;
de préférence dans laquelle l'étape a) comprend en outre l'addition d'un deuxième agent gélifiant, et l'étape a) est une étape pour ajouter le premier agent gélifiant et le deuxième agent gélifiant au plastifiant, les agiter jusqu'à homogénéité, après quoi le mélange est ajouté dans de l'eau et le tout est chauffé à une température comprise entre 60 et 98 °C sous agitation jusqu'à ce que le premier agent gélifiant et le deuxième agent gélifiant se dissolvent.

11. Enveloppe de capsule molle selon l'une quelconque des revendications 1 à 7 ou capsule molle selon la revendication 8 pour une utilisation dans des produits alimentaires, des produits nutraceutiques et des produits médicamenteux,
